# EUROPEAN PATENT SPECIFICATION

(11) **EP 0 964 047 B1**
(45) Date of publication and mention of the grant of the patent: **29.10.2003**
(21) Application number: 99304191.2
(22) Date of filing: 28.05.1999
(51) Int. Cl.: C09K 15/06, A61K 7/48

(54) **Anti-oxidant system**
Antioxydationssystem
Système anti-oxydant

(30) Priority: 01.06.1998 US 88466
(43) Date of publication of application: 15.12.1999
(73) Proprietor: Johnson & Johnson Consumer Companies, Inc., Skillman, New Jersey 08558-9418 (US)
(72) Inventor: Latoga, Gerard A., Ayala Homes, Mandaluyong City (PH); Dee, Kennie U., Pasig, Metro, Manila (PH)
(74) Representative: Mercer, Christopher Paul

(56) References cited:
- EP-A- 0 306 904
- EP-A- 0 825 191
- US-A- 5 114 716
- US-A- 5 468 474
- US-A- 5 607 921
- PATENT ABSTRACTS OF JAPAN vol. 012, no. 005 (C-467), 8 January 1988 (1988-01-08) & JP 62 164609 A (KANEBO LTD), 21 July 1987 (1987-07-21)
- CHEMICAL ABSTRACTS, vol. 113, no. 2, 9 July 1990 (1990-07-09) Columbus, Ohio, US; abstract no. 11917, TAGAWA, MASATO ET AL: "The application of magnesium ascorbate phosphate to skin care cosmetics" XP002111613 & J. SCCJ (1989), 23(3), 200-6 ,
- PATENT ABSTRACTS OF JAPAN vol. 014, no. 057 (C-0684), 2 February 1990 (1990-02-02) & JP 01 283208 A (NONOGAWA SHOJI:KK), 14 November 1989 (1989-11-14)

## Description

### FIELD OF THE INVENTION

This invention relates to fatty acid-based solid soap compositions, which are resistant to rancidity. Rancidity in such products can result in discoloration and malodor.

### BACKGROUND OF THE INVENTION

A common problem with products which contain fatty acids, such as soaps, is their susceptibility to rancidity. Rancidity can lead to discoloration or off-odor, and consequently, reduced shelf life. Rancidity typically develops because of auto-oxidation of fatty materials by atmospheric oxygen and is influenced by a number of factors, including light, heat, the presence of heavy metal ions, and the fatty acids themselves.

Certain ingredients in products such as soaps can accelerate the development of rancidity. For example, phenol-group containing compounds, such as anti-microbial agents like Triclosan, give rise to free radicals due to ionization, and these free radicals propagate the auto-oxidation reaction. The alkaline pH of soap formulations promotes the formation of free Triclosan radicals by ionization. Fragrances, heavy metal ions, light, and heat can also promote auto-oxidation. Soap recycle can also contribute to auto-oxidation.

Anti-oxidant systems have been suggested for use in soaps. For example, U.S. Patent No. 5,114,716 discloses an anti-oxidant system for stabilizing fatty acids and cosmetic compositions. This system includes at least one stabilized ascorbyl ester, at least one tocopherol or caffeic acid, a complexing agent, and a non-thiol polypeptide. However, such anti-oxidant systems are not satisfactory when the soap includes anti-microbial agents.

The present inventors have discovered that an anti-oxidant system that combines a water soluble, stable metal phosphate ester of vitamin C and a stable vitamin E ester, is effective as an anti-oxidant system for fatty acid-based compositions, such as cosmetics or personal care products, which contain phenol group-containing compounds.

### SUMMARY OF THE INVENTION

According to the present invention, there is provided a fatty acid-based solid soap composition as defined in the appendant claims.

### DETAILED DESCRIPTION OF THE INVENTION

The compositions of the present invention are non-opaque, transparent or translucent solid soaps, and include a neutralized fatty acid; a phenol group-containing compound, preferably an anti-microbial agent; and the specified anti-oxidant system to prevent or inhibit auto-oxidation which can result in rancidity.

The fatty acids useful in the products of the present invention are preferably fatty acids which are either linear or branched. Saturated fatty acids are preferred because they impart better color stability than do unsaturated fatty acids.

Single fatty acids or mixtures of two or more fatty acids may be used. Preferred fatty acids are C₁₂ - C₂₀ fatty acids. Examples of such fatty acids include, but are not limited to, vegetable-derived fatty acids such as lauric acid, myristic acid, palmitic acid, stearic acid, and isostearic acid. One non-limiting example of a mixture of fatty acids useful herein is a mixture of about 4% C₁₂-C₁₄ fatty acid, about 46% C₁₆ fatty acid, about 49% C₁₈ fatty acid, and about 1% C₁₈₋₁ fatty acid, based upon 100% total weight of fatty acids. Another non-limiting example is a mixture of about 2.5% C₁₂ - C₁₄ fatty acid, about 10% branched C₁₆ fatty acid, about 6% linear C₁₈ fatty acid, about 65% branched C₁₈ fatty acid, about 2% linear C₁₈ fatty acid, about 2.5% C₁₈₋₁ fatty acid, about 8% branched C₂₀ fatty acid, and about 4% C₂₂ fatty acid, based upon 100% total weight of fatty acids.

The fatty acid is formulated for use in the products of the present invention by neutralization with, for example, caustic soda, alkanolamine, or the like. A preferred alkanolamine is triethanolamine which is available as an 85%-95% solution. Combinations of neutralizing agents may be used, and neutralization by a dual alkali system is preferred.

Excess triethanolamine can be added to enhance transparency. Such an excess amount is typically a clarity enhancing effective amount.

Phenolic compounds are phenol group-containing compounds, and those useful herein include, but are not limited to, phenolic anti-microbial agents such as, for example, Triclosan; phenolic hydroxyacids such as, for example, salicylic acid; and salts of such acids. Triclosan is 5-chloro-2-(2,4-dichlorophenoxy) phenol. Triclosan tends to shorten significantly the shelf life of soaps and other products by accelerating rancidity and discoloration at levels typically used in personal care products. Useful phenolic compounds also include, but are not limited to, phenolic compounds which are encapsulated by, for example, liposomes. The amount of anti-microbial agent present in the soaps of the present invention may vary but is typically an anti-microbially effective amount. Preferred amounts of phenol group-containing compound or anti-microbial agent range, by weight, from about 0.1% to about 1%, based upon 100% of the total weight of the composition.

The anti-oxidant system of the present invention prevents or inhibits auto-oxidation. Accordingly, shelf life of said soap can be increased. Without being bound by any theory, it is believed that the anti-oxidant system converts free radicals to either their original structure or to non-reactive molecules, thereby retarding the auto-oxidation reaction.

The anti-oxidant system of the present invention is a two component system. The first component of the anti-oxidant system is vitamin E (tocopherol) or a water soluble ester thereof. A preferred ester is alpha-tocopherol acetate. The second component of the anti-oxidant system is C-2 or C-3 substituted vitamin C ester. Vitamin C and many of its derivatives are unstable and discolor when exposed to heat, light, or atmospheric oxygen. Most vitamin C derivatives are substituted at the C-6 position. However, C-6 substituted ascorbic acid derivatives can be oxidized to dehydro ascorbic acid by giving up the hydrogen ions from the hydroxyl group in the C-2 and C-3 positions. This results in discoloration. Additionally, most vitamin C derivatives are easily oxidized in aqueous solutions to produce yellow solutions. However, oxidation to dehydro ascorbic acid does not occur with C-2 or C-3 substituted ascorbic acid derivatives. This is most likely because the molecules can only give up one hydrogen ion.

Preferred C-2 or C-3 substituted vitamin C esters for use herein include, but are not limited to, phosphate or palmitate esters. Such esters include metal esters including, but are not limited to, magnesium ascorbyl phosphate, sodium ascorbyl phosphate, magnesium ascorbyl palmitate, sodium ascorbyl palmitate, and the like.

This anti-oxidant combination provides superior storage stability to solid, non-opaque soap formulations which include a phenol group-containing anti-microbial agent such as Trisclosan.

The amount of anti-oxidant system useful herein may vary, but will typically be an anti-oxidant effective amount. Preferably, the vitamin E or water soluble vitamin E ester component of the anti-oxidant system comprises less than or about 1% by weight, based upon 100% total weight of the composition. Most preferably, this component ranges from about 0.05 to about 0.1% by weight on the same basis. Preferably, the C-2 or C-3 substituted vitamin C ester component of the anti-oxidant system comprises less than or about 0.25% by weight, based upon 100% total weight of the composition. Most preferably, this component ranges from about 0.01 to about 0.05% by weight, on the same basis.

These levels of anti-oxidant system can increase the protection of the products of the present invention against rancidity for up to at least sixteen weeks. Excessive amounts of magnesium or sodium ascorbyl phosphate may yield a pro-oxidant effect, in that protection against auto-oxidation still is present, but the length of protection is decreased with increasing ascorbyl phosphate levels.

Additional components typically used in the preparation of soaps may be added. Such additives include, but are not limited to, UV absorbers to prevent photodegradation such as, for example, benzophenones such as benzophenone-3 (which can also have an anti-oxidant effect); humectants, such as glycerin; foam stabilizers, including, but not limited to, non-ionic foam stabilizers such as cocodiethanolamide; metal chelating agents including, but not limited to, disodium ethylene diamine tetra acetic acid (disodium EDTA); purified or deionized water; fragrances; colorants; or any combination of any of the foregoing.

The compositions of the present invention can be prepared by melting the fatty acid(s) at a temperature from about 55° C to about 60° C. The melt may then be pretreated with sodium metal-sulfite to improve color. The resultant melt is then neutralized with alkaline such as a preblended solution of triethanolamine and sodium hydroxide (50% solution) at appropriate stoichiometric proportions with excess triethanolamine for transparency.

The phenol group-containing compound(s) or anti-microbial agent(s) and anti-oxidant(s) are then added. Additives such as glycerin, DTPA, fragrances, colorants, and a UV absorber are also added as soon as neutralization is complete.

The molten product is then hot-poured into suitable trays and allowed to cool at ambient temperatures. When the desired bar hardening is achieved, the bars are cut, stamped, and wrapped.

### DESCRIPTION OF THE PREFERRED EMBODIMENTS

The following examples illustrate the invention without limitation.

### Example 1

A base clear soap mixture was prepared from the mixture of Table 1 below, using a Caframo mixer (Model RZR50) and a hot plate for heating. A combination of 0.1% by weight of α-tocopheryl acetate, 0.01% by weight of magnesium ascorbyl phosphate, 0.25% by weight of Triclosan, and 0.1% by weight of benzophenone-3, based upon 100% by weight of total composition, was added to the base soap, and this product was formed into bars.

**Table 1**

| Fatty Acid | % by Weight Based upon 100% Total Weight of Fatty Acids |
|---|---|
| Lauric Acid | 13.61 |
| Myristic Acid | 6.63 |
| Palmitic Acid | 8.90 |
| Stearic Acid | 8.90 |
| Isostearic Acid | 4.19 |
| Sodium Hydroxide | 4.17 |
| Triethanolamine | 40.48 |
| Glycerin | 1.00 |
| Coconut Diethanol-amine | 1.00 |
| EDTA | 0.16 |
| Fragrance RB 1001 | 0.20 |
| Water | q.s. |

### Comparative Example 1A

The procedure of Example 1 was followed omitting the tocopheryl acetate, magnesium ascorbyl phosphate, and benzophenone-3.

### Comparative Example 1B

The procedure of Example 1 was followed omitting the tocopheryl acetate and the magnesium ascorbyl phosphate.

The bars of Example 1 discolored four times later than the bars of Comparative Example 1 and two times later than the bars of Comparative Example 1B, when stored at 50°C.

### Example 2

The procedure of Example 1 was followed substituting sodium ascorbyl phosphate for the magnesium ascorbyl phosphate.

### Example 3

The procedure of Example 1 was followed substituting 0.05% by weight of tocopheryl acetate for the amount of tocopheryl acetate.

### Example 4

A mixture of fatty acids as shown in Table 2 below, is melted at a temperature between 55°C and 65°C.

**Table 2**

| Fatty Acid | % by Weight Based upon 100% Total Weight of Fatty Acids |
|---|---|
| C₁₂ - C₁₄ | 2.5 |
| C₁₆ Branched | 10 |
| C₁₆ Linear | 6 |
| C₁₀ Branched | 65 |
| C₁₈ Linear | 2 |
| C₁₈₋₁ | 2.5 |
| C₂₀ Branched | 8 |
| C₂₂ | 4 |

Sodium metabisulfite is added to the melt. The melt is then neutralized with a solution of triethanolamine and sodium hydroxide with an excess of triethanolamine. Triclosan, and an anti-oxidant system of alpha-tocopherol acetate and magnesium ascorbyl phosphate are added after neutralization.

The melt is hot poured into molds and is allowed to cool to a solid transparent bar.

### Example 5

The method of Example 4 is followed substituting the fatty acid mixture shown in Table 3 below, for the fatty acid mixture of Table 2.

**Table 3**

| Fatty Acid | % by Weight Based upon 100% Total Weight of Fatty Acids |
|---|---|
| C₁₂ - C₁₄ | 4 |
| C₁₈ | 46 |
| C₁₈ | 49 |
| C₁₈₋₁ | 1 |

Many obvious variations of the present invention within the scope of the appended claims will suggest themselves to those skilled in the art in light of the above, detailed description.

## Claims

1. A fatty acid-based solid soap composition comprising:
(a) at least one neutralized fatty acid;
(b) a phenol group-containing compound; and
(c) an anti-oxidant system comprising (i) vitamin E or a water-soluble ester thereof and (ii) a C-2 or C-3 substituted vitamin C ester:
wherein said solid soap composition is non opaque, translucent or transparent.

2. The system of claim 1, wherein said water-soluble vitamin E ester comprises alpha-tocopheryl acetate.

3. The system of claim 1 or 2, wherein said C-2 or C-3 substituted vitamin C ester comprises a phosphate ester.

4. The system of claim 1 or claim 2, wherein said C-2 or C-3 substituted vitamin C ester is magnesium ascorbyl phosphate, sodium ascorbyl phosphite, magnesium ascorbyl palmitate, sodium ascorbyl palmitate or any combination of any of the foregoing.

5. The composition of any one of claims I to 4, wherein said fatty acid is a C₁₂ - C₂₀ linear fatty acid, a C₁₂ - C₂₀ branched fatty acid, or any combination thereof and preferably is lauric acid, myristic acid, palmitic acid, stearic acid, isostearic acid or any combination of any of the foregoing.

6. The composition of claim 5, wherein said phenol group-containing compound comprises an anti-microbial agent which preferably is Triclosan.

7. The composition of claim 5 or claim 6, wherein said anti-oxidant system comprises less than or about 1.25% by weight, based upon 100% total weight of said composition.

8. The composition of any one of claims 5 to 7, further comprising (d) an alkanolamine, which preferably is triethanolamine.

## Patentansprüche

1. Feste Seifen-Zusammensetzung auf Fettsäure-Basis, umfassend:
(a) mindestens eine neutralisierte Fettsäure;
(b) eine Phenolgruppe-enthaltende Verbindung; und
(c) ein Antioxidationsmittel-System, umfassend (i) Vitamin E oder einen wasserlöslichen Ester davon und (ii) einen C-2- oder C-3-substituierten Vitamin C-Ester;
wobei die feste Seifen-Zusammensetzung nicht opak, transluzent oder transparent ist.

2. System nach Anspruch 1, wobei der wasserlösliche Vitamin E-Ester alpha-Tocopherylacetat umfaßt.

3. System nach Anspruch 1 oder 2, wobei der C-2- oder C-3-substituierte Vitamin C-Ester einen Phosphatester umfaßt.

4. System nach Anspruch 1 oder 2, wobei der C-2- oder C-3-substituierte Vitamin C-Ester Magnesiumascorbylphosphat, Natriumascorbylphosphit, Magnesiumascorbylpalmitat, Natriumascorbylpalmitat oder jede Kombination aus einer der vorangehenden Verbindungen ist.

5. Zusammensetzung nach einem der Ansprüche 1 bis 4, wobei die Fettsäure eine lineare C₁₂-C₂₀-Fettsäure, eine verzweigtkettige C₁₂-C₂₀-Fettsäure oder jede Kombination davon ist, und vorzugsweise Laurinsäure, Myristinsäure, Palmitinsäure, Stearinsäure, Isostearinsäure oder jede Kombination aus einer der vorangehenden Verbindungen ist.

6. Zusammensetzung nach Anspruch 5, wobei die Phenolgruppe-enthaltende Verbindung ein antimikrobielles Agens umfaßt, das vorzugsweise Triclosan ist.

7. Zusammensetzung nach Anspruch 5 oder 6, wobei das Antioxidationsmittel-System weniger als oder ungefähr 1,25 Gew.-%, bezogen auf 100% Gesamtgewicht der Zusammensetzung, umfaßt.

8. Zusammensetzung nach einem der Ansprüche 5 bis 7, die weiterhin (d) ein Alkanolamin umfaßt, das vorzugsweise Triethanolamin ist.

## Revendications

1. Composition de savon solide à base d'acide gras comprenant :
(a) au moins un acide gras neutralisé ;
(b) un composé contenant un groupe phénol ; et
(c) un système anti-oxydant comprenant (i) de la vitamine E ou un de ses esters solubles dans l'eau et (ii) un ester de vitamine C substitué en C-2 ou C-3 ; dans laquelle ladite composition de savon solide est non opaque, translucide ou transparente.

2. Système selon la revendication 1, dans lequel ledit ester de vitamine E soluble dans l'eau comprend l'acétate d'alpha-tocophéryle.

3. Système selon la revendication 1 ou 2, dans lequel ledit ester de vitamine C substitué en C-2 ou C-3 comprend un ester de phosphate.

4. Système selon la revendication 1 ou la revendication 2, dans lequel ledit ester de vitamine C substitué en C-2 ou C-3 est l'ascorbylphosphate de magnésium, l'ascorbylphosphite de sodium, l'ascorbylpalmitate de magnésium, l'ascorbylpalmitate de sodium ou toute combinaison de n'importe lesquels des précédents.

5. Composition selon l'une quelconque des revendications 1 à 4, dans laquelle ledit acide gras est un acide gras linéaire en C₁₂-C₂₀, un acide gras ramifié en C₁₂-C₂₀, ou toute combinaison de ceux-ci et est de préférence l'acide laurique, l'acide myristique, l'acide palmitique, l'acide stéarique, l'acide isostéarique, ou toute combinaison de n'importe lesquels des précédents.

6. Composition selon la revendication 5, dans laquelle ledit composé contenant un groupe phénol comprend un agent antimicrobien qui est de préférence le Triclosan.

7. Composition selon la revendication 5 ou la revendication 6, dans laquelle ledit système anti-oxydant constitue moins de ou environ 1,25 % en poids, rapporté à 100 % de poids total de ladite composition.

8. Composition selon l'une quelconque des revendications 5 à 7, comprenant en outre (d) une alcanolamine, qui est de préférence la triéthanolamine.
